# FASCICULE DE BREVET EUROPEEN

(11) **EP 3 060 110 B1**
(45) Date de publication et mention de la délivrance du brevet: **01.12.2021**
(21) Numéro de dépôt: 14824890.9
(22) Date de dépôt: 24.10.2014
(51) Int. Cl.: A61B 5/12, A61B 5/246, A61B 5/38, A61B 5/00, H04R 25/00

(54) **MÉTHODE ÉLECTROPHYSIOLOGIQUE D'ÉVALUATION DE L'EFFICACITÉ D'UNE PROTHÈSE AUDITIVE**
ELEKTROPHYSIOLOGISCHES VERFAHREN ZUR BEWERTUNG DER EFFEKTIVITÄT EINES HÖRGERÄTS
ELECTROPHYSIOLOGICAL METHOD FOR ASSESSING THE EFFECTIVENESS OF A HEARING AID

(30) Priorité: 25.10.2013 FR 1360447
(43) Date de publication de la demande: 31.08.2016
(73) Titulaire: Hospices Civils De Lyon, 69229 Lyon Cedex 02 (FR); Université Claude Bernard Lyon I, 69622 Villeurbanne Cedex (FR)
(72) Inventeur: THAI-VAN, Hung, F-69003 Lyon (FR); CACLIN, Anne, F-38300 Serezin de La Tour (FR); BELLIER, Ludovic, F-69003 Lyon (FR); VESSON, Jean-François, F-69340 Francheville (FR); VEUILLET, Evelyne, F-69004 Lyon (FR)
(74) Mandataire: Cabinet Beau de Loménie
(86) Numéro de dépôt international: PCT/FR2014/052711
(87) Numéro de publication internationale: WO 2015/059425

(56) Documents cités:
- WO-A1-2013/017169
- US-B2- 8 014 853
- HARVEY DILLON: "So, baby, how does it sound? Cortical assessment of infants with hearing aids", THE HEARING JOURNAL, vol. 58, no. 10, 1 octobre 2005 (2005-10-01), pages 10-17, XP055125165, ISSN: 0745-7472, DOI: 10.1097/01.HJ.0000285781.30125.64
- KRAUS N ET AL: "The mismatch negativity cortical evoked potential elicited by speech in cochlear-implant users", HEARING RESEARCH, ELSEVIER SCIENCE PUBLISHERS, AMSTERDAM, NL, vol. 65, no. 1-2, 1 février 1993 (1993-02-01), pages 118-124, XP024397053, ISSN: 0378-5955, DOI: 10.1016/0378-5955(93)90206-G [extrait le 1993-02-01]
- None

## Description

La présente invention concerne le domaine technique de l'évaluation des prothèses auditives. L'invention concerne d'une part des prothèses auditives externes telles que des contours d'oreille conventionnels, des contours d'oreille à tubes fins (par exemple, « Life Tubes »), des contours d'oreille à écouteur déporté dans le conduit auditif, des prothèses intra-auriculaires ou des lunettes auditives. L'invention concerne également des prothèses totalement ou partiellement implantées telles que des implants à ancrage osseux, des implants d'oreille moyenne semi- ou totalement implantables (à conduction aérienne ou à conduction osseuse), des implants cochléaires, des implants cochléaires électro-acoustiques ou des implants du tronc cérébral.

La surdité étant un état pathologique caractérisé par une perte partielle ou totale du sens de l'ouïe, la prothèse a pour but de pallier cette perte du sens de l'ouïe.

Actuellement, le réglage des systèmes de correction auditive en vue de l'amélioration de la compréhension de la parole repose, dans la pratique clinique, sur des tests subjectifs nécessitant la participation active du sujet malentendant (audiométries tonale et vocale) et sur l'expertise de l'audioprothésiste. Sachant que les prothèses auditives disponibles sur le marché sont des dispositifs biomédicaux coûteux et compte tenu de l'impact de la surdité sur les capacités de communication et de socialisation à tous les âges de la vie, il devient crucial de pouvoir régler de manière optimale les prothèses auditives afin de garantir la meilleure compliance thérapeutique. Il existe notamment des situations où la fiabilité et la reproductibilité des tests subjectifs posent question (âges extrêmes : nourrisson et quatrième âge, handicaps associés, Trisomie 21, par exemple) et pour lesquelles le recours à une méthode objective de réglage et de suivi de l'appareillage auditif devrait être recommandé. Aujourd'hui, en l'absence de port d'une prothèse auditive, des mesures électrophysiologfques permettent l'évaluation objective de la fonction auditive en routine clinique.

D'une manière classique, en réponse à un stimulus de parole, on distingue deux types de réponse :
- des réponses de haute fréquence (au-delà d'environ 80 Hz), dont l'origine est supposée être sous-corticale (provenant notamment du tronc cérébral), et donc généralement appelées dans la littérature « Speech ABR » (Speech Auditory Brainstem Response). Les caractéristiques physiques des « speech ABR » permettent non seulement de vérifier que le son de parole délivré est détecté par le système nerveux auditif, mais également qu'il est correctement codé et que ce codage permet au système nerveux auditif de le discriminer d'un autre son de parole ;
- des réponses de basse fréquence (en-dessous d'environ 80 Hz), dont l'origine est supposée être essentiellement corticale. Ces réponses sont généralement labellisées par leur signe et leur latence en millisecondes au vertex : P50, N100, P200, etc.

La demande de brevet WO 2008/116462 décrit, chez le sujet porteur d'une prothèse auditive, un système de recueil de réponses électrophysiologiques à des sons simples (clics, bouffées tonales), synthétisés avec un générateur de signaux électriques conventionnel. Les sons étant présentés à un individu *via* sa prothèse auditive, le système se propose de résoudre le problème de la synchronisation entre le signal acoustique et le système de mesure neurophysiologique. De plus, le système revendique la capacité à tester le seuil de détection auditif permis par le port de la prothèse. Néanmoins, cette demande de brevet ne décrit pas d'utilisation de sons langagiers comme stimulus, ni d'amélioration du réglage de la prothèse reposant sur l'évaluation de la discrimination neurophysiologique des sons de parole. Or, la capacité à détecter un son ne préjuge en rien de la capacité du sujet à le discriminer d'un autre son proche mais présentant une différence acoustique pertinente pour le système nerveux central auditif.

Le document HARVEY DILLON « So, baby how does it sound ? Cortical assessment of infants with hearing aids », THE HEARING JOURNAL, vol. 58, n° 10, 1 Octobre 2005 (2005-10-01), pages 10-17, XP055125165, ISSN : 0745-7472, DOI : 10.1097/01.HJ.0000285781.30125.64 est une interview d'un médecin décrivant une méthode visant à régler les prothèses auditives chez l'enfant. Cette méthode vise à émettre un stimulus au travers de la prothèse auditive et à recueillir les Potentiels Evoqués Auditifs corticaux. La mesure des réponses corticales n'apporte pas une information suffisamment fine sur la représentation d'un son dans le système nerveux auditif. Par ailleurs, le choix des stimulations proposées ne permet pas d'apprécier la capacité d'un sujet à discriminer un son d'un autre son proche mais présentant une différence acoustique pertinente pour le système nerveux central auditif.

Le document KRAUS N ET AL « The mismatch negativity cortical evoked potential elicited by speech in cochlear-implant users », HEARING RESEARCH, ELSEVIER SCIENCE PUBLISHERS, AMSTERDAM, NL, vol.65, n° 1-2, 1 février 1993 (1993-02-01), pages 118-124, XP024397053, ISSN : 0378-5955, DOI : 10.1016/0378-59555(93)90206-G décrit une étude visant à montrer l'existence d'une différentiation de deux sons différents par le système auditif de patients équipés d'un implant cochléaire. La méthode décrite vise à recueillir les Potentiels Evoqués Auditifs corticaux de sorte qu'une telle mesure n'apporte pas une information suffisamment précise sur la représentation d'un son dans le système nerveux auditif.

Le brevet US 8 014 853 décrit, chez le sujet ne portant pas de prothèse auditive, un système de recueil des réponses électrophysiologlques auditives du tronc cérébral à des sons de parole de type syllabe [consonne-voyelle] (« speech Auditory Brainstem Responses »). Ces réponses électrophyslologlques peuvent ensuite être analysées et comparées à une base de données normatives pour aider au diagnostic d'un trouble du traitement auditif par le système nerveux central auditif. Si le diagnostic est avéré, l'invention permet de poser l'indication d'une intervention thérapeutique.

Dans ce contexte, il devient pertinent de vouloir vérifier la qualité du traitement neurophysiologique des sons de parole chez l'individu porteur d'une prothèse auditive, sans nécessairement faire appel à des données normatives. En effet, pour que le traitement des sons de parole par le système nerveux central auditif conduise à une bonne compréhension du langage, l'élément déterminant n'est ni la forme exacte de la réponse neurophysiologique à une stimulation donnée, ni sa ressemblance avec des données normatives. C'est surtout la capacité du système nerveux central à traiter différemment des sons de parole, acoustiquement proches, qui permet à un individu porteur d'une prothèse auditive de bien comprendre le locuteur.

Le but de l'invention est donc de proposer une méthode objective d'évaluation de l'efficacité d'une prothèse auditive, considérant que le réglage de cette prothèse doit permettre à un individu appareillé de discriminer les principaux traits acoustiques de la parole et donc de distinguer au moins deux sons de parole acoustiquement proches.

Ainsi, l'objet de l'invention est une méthode d'évaluation de l'efficacité d'une prothèse auditive telle que définie par la revendication 1.

Le demandeur entend ainsi répondre à un besoin avéré mais n'ayant pas de solution en pratique. En effet, la discrimination neurophysiologique entre deux sons langagiers acoustiquement proches est déterminante dans la compréhension du langage. De plus, l'invention permet de tester soit une seule prothèse (stimulation monaurale), soit deux prothèses l'une après l'autre (stimulation monaurale, droite puis gauche, ou inversement), soit deux prothèses simultanément (stimulation binaurale, stimulation dichotique). L'invention permet d'optimiser la stéréophonie permise par un appareillage auditif bilatéral, la mono-stéréophonie permise par un appareillage unilatéral en présence d'une audition controlatérale subnormale, ou la stéréo-acousie résultant d'un appareillage unilatéral en présence d'une audition controlatérale anormale. Les signaux neurophysiologiques émis par ledit système nerveux auditif en réponse au stimulus auditif et enregistrés au moyen d'un système d'électro- ou de magnéto-encéphalographie seront désignés ci-après par signaux électrophysiologiques.

Pour un individu atteint de surdité, l'important n'est pas de se conformer à des données normatives mais plutôt de « recouvrer » l'ouïe ou tout du moins d'être capable de distinguer différentes unités linguistiques qui constituent son langage pour qu'il réapprenne à comprendre ce langage et à entrer en interaction avec différents locuteurs.

Les mesures électrophysiologiques permettent d'objectiver si le signal acoustique de parole est correctement transmis par la prothèse auditive et traité par les voies auditives sous-corticales et corticales. Pour cela, l'invention permet le recueil simultané des réponses du tronc cérébral, encore appelées sous-corticales (« speech Auditory Brainstem Responses » désignées ci-après « speech ABR »), et des réponses corticales (« Potentiels Evoqués Auditifs corticaux » désignés ci-après «**PEA**» corticaux) aux sons de parole amplifiés par la prothèse auditive. Ce recueil se fait chez le patient appareillé en situation écologique d'écoute, c'est-à-dire *via* ses propres appareils auditifs ou ceux qu'il pourrait acquérir. Les réponses évoquées par les sons de parole sont dans le détail les suivantes :
- au niveau sous-cortical, une réponse transitoire impulsionnelle (« onset response ») déclenchée par le début du stimulus et similaire aux réponses du tronc cérébral à un stimulus transitoire de type clic, suivie d'une réponse soutenue à la fréquence (FFR : « Frequency Following Response »), cette dernière étant décrite comme reflétant l'activité neuronale composite du tronc cérébral synchronisée avec la périodicité du stimulus ;
- et au niveau cortical, une succession d'ondes plus lentes que celles générées au niveau sous-cortical, notamment sur les électrodes fronto-centrales (onde P50, suivie du complexe N1-P2-N2 également appelé N100-P200-N200) et sur les électrodes temporales (complexe T).

Jusqu'à ce jour :
- le recueil de speech ABR chez des sujets appareillés auditivement n'a été fait qu'oreilles nues (c'est-à-dire après avoir enlevé les prothèses), jamais en administrant des sons de parole *via* la prothèse auditive ;
- l'enregistrement simultané de speech ABR et de «**PEA**» corticaux n'a pas été proposé chez les porteurs de prothèse auditive.

La méthode consiste à comparer les signaux électrophysiologlques correspondant aux différentes unités linguistiques testées en effectuant une ou plusieurs des étapes suivantes, consistant :
- à extraire des latences de pics, des amplitudes de pics, des différences de latence entre pics, des pentes de la courbe entre deux pics, ou des aires sous une partie de la courbe ;
- à rechercher dans des formes d'onde et/ou des décompositions temps-fréquence, une réponse attendue spécifiquement pour l'une desdites unités linguistiques ;
- à comparer directement des formes d'ondes de chaque signal neurophysiologique recueilli en réponse par exemple par soustraction ;
- à comparer des décompositions temps-fréquence.

L'avantage de la mise en œuvre de ces différentes étapes réside dans le fait qu'elles sont faciles à utiliser pour toute personne familière avec le traitement du signal. Ces comparaisons s'effectuent après traitement du signai brut, traitement comportant une ou plusieurs des étapes suivantes : le rejet et/ou la correction d'artefact, le filtrage du signal, la moyenne de tous les signaux en réponse à toutes les présentations d'un même stimulus, et le passage du domaine temporel au domaine spectral. Ce sont des étapes classiques constituant la base du traitement du signal en électrophysiologie humaine. On peut également y ajouter des méthodes avancées de débruitage des signaux et/ou de séparation de sources, comme l'analyse en composantes principales ou l'analyse en composantes indépendantes connues de l'homme du métier.

Selon un mode préférentiel de mise en œuvre, la méthode consiste à choisir les unités linguistiques parmi les phonèmes, tonèmes ou chronèmes, ou leurs assemblages en syllabes.

L'avantage de choisir les unités linguistiques parmi les phonèmes, tonèmes ou chronèmes réside dans le fait que ceux-ci couvrent l'ensemble des langues parlées. Les unités linguistiques se distinguent les unes des autres par différents traits acoustiques. Des exemples de traits acoustiques permettant de discriminer les phonèmes du français sont : soit un contenu fréquentiel du type grave/aigu, compact/diffus ou oral/nasal, soit une caractéristique temporelle du type continu/discontinu, vocalique/non vocalique ou sonore (ou voisé)/sourd, soit encore une combinaison de ces traits.

Dans les langues tonales, comme le Chinois mandarin, on pourra utiliser comme unité linguistique des caractères ou mots, chaque caractère pouvant être constitué : d'une partie initiale telle qu'une consonne et d'une partie finale telle qu'une voyelle.

Les mots monosyllabiques appelés aussi « caractères » sont constitués d'une partie initiale et d'une partie finale, et sont différenciés par des variations tonales. Par exemple, quatre variations tonales sont utilisées en Chinois mandarin dans le cas de la voyelle /a/ :
- ã, une tonalité stable pendant toute la prononciation du /a/ ;
- á, une tonalité montante entre le début de la prononciation et la fin du /a/ ;
- à, une tonalité descendante entre le début de la prononciation et la fin du /a/, et
- ǎ, une tonalité descendante en début de prononciation puis remontante en fin de prononciation.

Selon un mode préférentiel de mise en œuvre, la méthode consiste à choisir les unités linguistiques parmi les paires minimales (deux phonèmes distingués par un seul trait acoustique), notamment dans une matrice de confusion phonétique.

Selon un mode préférentiel de mise en œuvre, la méthode consiste à identifier, en fonction du résultat dudit traitement des signaux, au moins un trait acoustique dudit stimulus dont le traitement par ledit système nerveux auditif est insuffisant pour permettre ladite discrimination neurophysiologique desdites unités linguistiques émises.

Selon un mode préférentiel de mise en œuvre, la méthode consiste à changer au moins un paramètre de réglage de ladite prothèse auditive en fonction dudit trait acoustique, dont le traitement par ledit système nerveux auditif est insuffisant pour permettre ladite discrimination neurophysiologique desdites unités linguistiques émises.

Selon un mode préférentiel de mise en œuvre, la méthode consiste à transmettre le stimulus à une prothèse par une liaison sans fil.

Diverses autres caractéristiques ressortent de la description faite ci-dessous en référence aux dessins annexés qui montrent, à titre d'exemples non limitatifs, des formes de réalisation de l'objet de l'invention :
La **Figure 1** représente schématiquement un dispositif expérimentai pour la mise en œuvre de la présente invention.
Les **Figures 2A, 2B, 2C** et **2D** représentent respectivement un stimulus auditif constitué de l'unité linguistique /ta/, sa transmission au moyen d'un écouteur de type insert, une mesure de speech ABR et une mesure de «PEA» corticaux.
Les **Figures 2E, 2F, 2G** et **2H** représentent respectivement un stimulus auditif constitué de l'unité linguistique /ta/, sa transmission au moyen d'une prothèse de type contour d'oreille, une mesure de speech ABR et une mesure de **«PEA»** corticaux.
Les **Figures 3A, 3B, 3C** et **3D** représentent respectivement un stimulus auditif constitué de l'unité linguistique /da/, sa transmission au moyen d'une prothèse de type contour d'oreille, une mesure de speech ABR et une mesure de «PEA» corticaux.
Les **Figures 3E, 3F, 3G** et **3H** représentent respectivement un stimulus auditif constitué de l'unité linguistique /ta/, sa transmission au moyen d'une prothèse de type contour d'oreille, une mesure de speech ABR et une mesure de **«PEA»** corticaux.
Les **Figures 4A, 4B** et **4C** représentent des étapes d'une mise en œuvre de la présente invention par soustraction des mesures neurophysiologiques obtenues pour les deux unités linguistiques /da/ et /ta/ présentées au moyen d'une prothèse de type contour d'oreille.
La **Figure 5** représente une matrice de confusion en anglais.
La **Figure 6** représente les contenus fréquentiels caractéristiques de chaque consonne en français (acougramme phonétique).

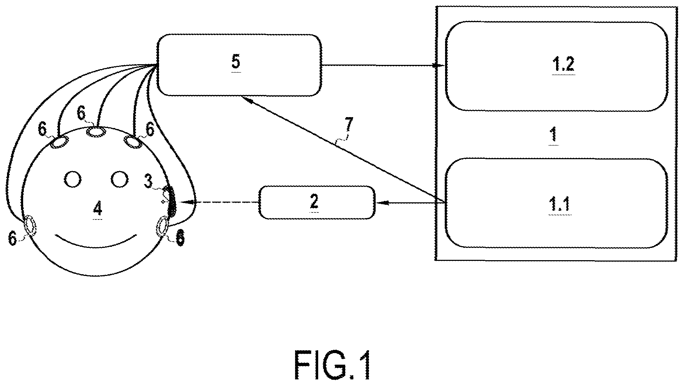

La **Fig. 1** représente un dispositif expérimental pour la mise en œuvre de l'invention. Un tel dispositif comporte à titre illustratif une unité centrale **1** telle qu'un ordinateur comportant d'une part un module de commande **1.1** et, d'autre part, un module d'enregistrement des données **1.2.** Le dispositif peut comporter deux ordinateurs séparés, un premier comprenant le module de commande **1.1** et un second comprenant le module d'enregistrement des données **1.2**.

En particulier, le module de commande **1.1** de l'unité centrale **1** est relié à un émetteur **2** adapté pour la transmission de signaux de commande vers une prothèse auditive **3**. Cette prothèse auditive **3** peut être du type des prothèses auditives externes : contours d'oreille conventionnels, contours d'oreille à tubes fins, contours d'oreille à écouteurs déportés, prothèses intra-auriculaires, lunettes auditives, ou bien des prothèses totalement ou partiellement implantées : implants à ancrage osseux, implants d'oreille moyenne semi- ou totalement implantables (à conduction aérienne ou à conduction osseuse), implants cochléaires, implants cochléaires électro-acoustiques, implants du tronc cérébral. La transmission de signaux entre l'émetteur **2** et la prothèse auditive **3** peut être de type liaison filaire ou sans fil, i.e. par transmission hertzienne (modulation de fréquence), transmission radioélectrique (exemple : Bluetooth^{®}) ou tout type de transmission par ondes électromagnétiques. La prothèse auditive **3** est destinée à être portée par un individu **4**. Ainsi, l'unité centrale **1** permet d'émettre un stimulus auditif au système nerveux auditif de l'individu **4** *via* la prothèse auditive **3**.

De plus, l'unité centrale **1** est reliée à un amplificateur électro- ou magnéto-encéphalographique **5** (noté ci-après amplificateur EG). Cet amplificateur EG **5** est relié à des électrodes pour l'électro-encéphalographie ou à des capteurs pour la magnéto-encéphalographie **6** disposés en contact avec ou à proximité de la tête de l'individu **4**. Ces électrodes ou capteurs **6** sont adaptés pour mesurer ou capter l'activité électrophysiologique spécifique dans le système nerveux central auditif de l'individu **4**. Ainsi, l'unité centrale **1** peut enregistrer cette activité électrophysiologique spécifique de type « speech ABR » ou réponse du tronc cérébral à un son de parole, ou de type « PEA » corticaux (réponses du cortex cérébral à un son de parole).

Enfin, l'unité centrale **1** est adaptée pour synchroniser l'enregistrement de l'activité électrophysiologique reçue *via* l'amplificateur EG **5** avec le stimulus auditif émis, *via* une connexion **7** de synchronisation entre le module de commande **1.1** et l'amplificateur EG **5**.

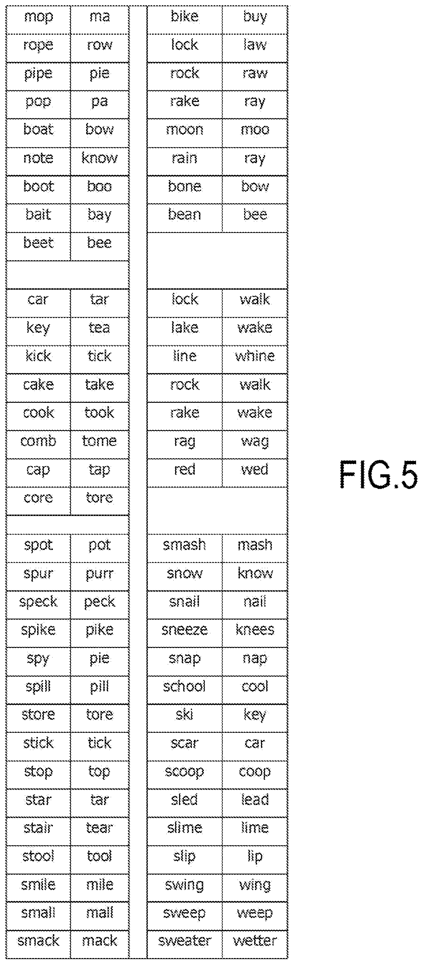

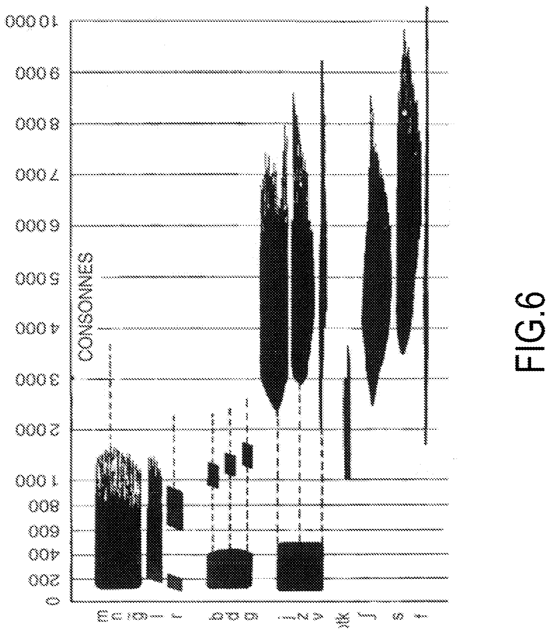

En particulier, selon l'invention, le stimulus auditif émis est du type d'une paire d'unités linguistiques choisies dans une matrice de confusion phonétique. On comprend par unité linguistique un phonème, un tonème ou un chronème, ou encore leur assemblage en syllabe. Par exemple, l'assemblage du phonème /t/ avec le phonème /a/ résulte en la syllabe de type consonne-voyelle /ta/. Les phonèmes, tonèmes et chronèmes sont connus comme les unités de base en phonétique, et permettent de constituer l'ensemble des mots formant les langues. Les matrices de confusion sont connues en phonologie et sont dépendantes de la langue **(****Fig. 5** et **6**). Elles ont été décrites dans la littérature aussi bien pour le français (Lefevre F., « Impact de la perte auditive sur la perception de la parole : confusions phonétiques », dans Précis d'Audioprothèse, Production, phonétique acoustique et perception de la parole, Elsevier Masson, 2008, pp. 389-399) que pour l'anglais (Miller G.A. & Nicely P.E., « An Analysls of Perceptual Confusions among Some English Consonants », Journal of the Acoustical Society of America, 1954, 27 (2), 338-352).

En français, pour comparer deux unités linguistiques, il peut suffire de choisir deux consonnes phonétiquement proches, prononcées avec la même voyelle, par exemple, les syllabes /ta/ et /da/.

Il est connu de l'homme du métier d'introduire un insert dans le conduit auditif d'un individu pour effectuer des mesures d'activité électrophysiologique spécifique. On comprend par insert un écouteur intra-auriculaire constitué d'un tube en plastique rempli d'air par lequel sont transmis des ondes sonores et d'un bouchon en mousse permettant son ajustement au diamètre du conduit auditif externe. De manière générale, l'homme du métier n'utilise pas de prothèse à la place de l'insert, car il préjuge de ne pas pouvoir faire de mesures de l'activité électrophyslologlque spécifique du tronc cérébral dans ces conditions.

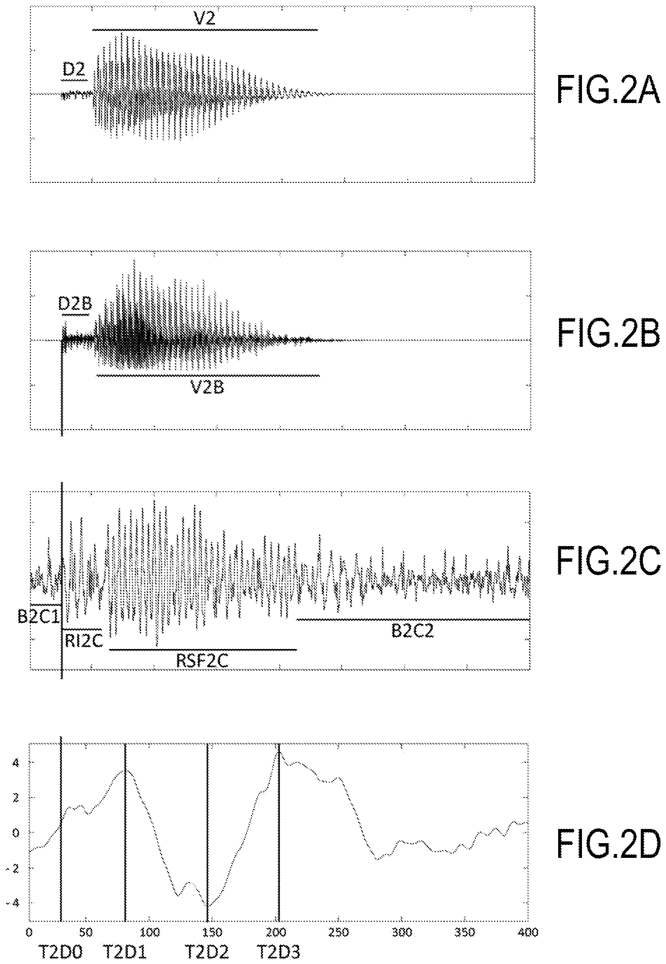

Les **Fig. 2A** à **2H** montrent la possibilité d'utiliser des prothèses auditives en lieu et place des inserts pour effectuer des mesures de l'activité électrophysiologique en réponse aux stimulus administrés *via* la prothèse auditive.

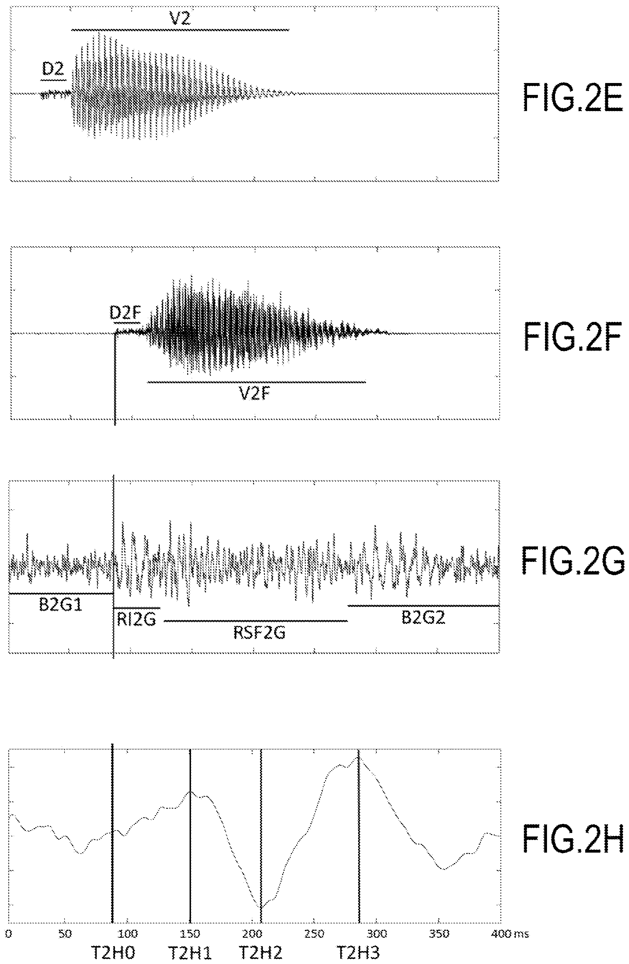

En effet, la syllabe /ta/ a été testée au moyen d'inserts **(****Fig. 2A** à **2D**), et de prothèses auditives **(****Fig. 2E** à **2H****)**.

La **Fig. 2A**, comme la **Fig. 2E**, représente la syllabe /ta/ telle qu'elle est émise par le module de commande **1.1** de l'unité centrale **1**. En particulier, cette syllabe comporte tout d'abord une partie transitoire de désocclusion, correspondant à l'expulsion de l'air contenu dans la cavité orale conséquente au retrait du bout de la langue des incisives supérieures. La désocclusion et la mise en vibration des cordes vocales pour produire la voyelle /a/ sont séparées de 25 ms, et forment les limites de la zone **D2** assimilable à la consonne /t/ (en y incluant la désocclusion et en excluant la mise en vibration des cordes vocales). Cette désocclusion **D2** est suivie d'une zone **V2** de voyelle /a/ de durée 200 ms. La zone **V2** comporte une pseudo-période, c'est-à-dire des ondulations ayant la même période mais des amplitudes variables. En particulier, ici, la pseudo-période est de durée 4,6 ms (ce qui correspond à une fréquence fondamentale de 218 Hz).

Tel que représenté à la **Fig. 2B****,** la syllabe émise par l'insert comporte une structure comparable à celle de la **Fig**. **2A**. La syllabe de la **Fig. 2B** comporte une zone **D2B** de consonne /t/ de durée 25 ms suivie d'une zone **V2B** de voyelle /a/ de durée 200 ms. La zone **V2B** a sensiblement la même durée que la zone **V2** et comporte la même pseudo-période. La voyelle retransmise par les inserts est assez fidèle au signal envoyé ; par contre, la consonne - et tout particulièrement la désocclusion - est très distordue : cela résulte en un large pic au tout début du signal en sortie des inserts, ce qui diffère grandement avec le signal envoyé. Cette déformation est imputable aux tubes en plastique flexibles des inserts, qui ont un effet de distorsion connu sur les signaux acoustiques.

La **Fig. 2F** représente le son de la syllabe /ta/ tel qu'il est émis par la prothèse auditive **3**. On remarque notamment un décalage temporel de 67 ms entre l'émission de la syllabe par le module de commande **1.1** de l'unité centrale **1** alors que le décalage temporel dû à l'insert est négligeable (moins de 1 ms avec des tubes en plastique flexibles d'une longueur de 30 cm et une vitesse du son dans l'air de 340 m/s).

De plus, la syllabe représentée en **Fig. 2F** comporte aussi une structure comparable à celle des **Fig. 2A** et **2E**. En effet, cette structure comprend notamment une zone de consonne **D2F** et une zone de voyelle **V2F**. La consonne est fidèle au signal envoyé, mais cette fois on observe une distorsion fréquentlelle de la voyelle. Le signal transmis dépend néanmoins des caractéristiques de la prothèse et des réglages que l'on décide de lui appliquer.

Les **Fig. 2C** et **2G** représentent respectivement les « speech ABR » mesurées à la suite de l'émission de la syllabe /ta/ dans les inserts et dans les prothèses auditives **3**. Ces mesures d'activité électrophyslologlque spécifique de type «speech ABR» ont été acquises avec un système « actiCHamp » (« BrainProducts ») à électrodes actives, à une fréquence d'échantillonnage de 25 kHz, avec une cadence de stimulation de trois unités linguistiques par seconde (stimulation binaurale, c'est-à-dire des deux oreilles simultanément). La polarité des stimulations auditives est alternée afin de s'affranchir par moyennage d'éventuels artéfacts de stimulation dans les réponses électrophysiologiques. Les données sont enregistrées à l'électrode Cz (placée au vertex), référencée à la moyenne de deux électrodes de recueil placées sur les mastoïdes. Les « speech ABR » sont obtenues à partir de 3000 essais élémentaires, après rejet d'artéfacts, moyennage des essais ne présentant pas d'artéfacts, et filtrage passe-bande entre 80 et 1000 Hz.

La **Fig. 2C** comporte deux zones de bruit **B2C1** et **B2C2,** une réponse impulslonnelle **RI2C** et une zone de réponse soutenue à la fréquence **RSF2C.** La réponse impulsionnelle **RI2C** est décelable, d'une part car son amplitude est supérieure à celle du bruit **B2C**, et d'autre part car elle se situe à une latence d'environ 6 ms après la désocclusion à l'origine de cette réponse, ce qui correspond au temps d'arrivée des informations auditives dans la partie supérieure du tronc cérébral. La zone de réponse soutenue à la fréquence **RSF2C** est décelable par le fait qu'elle dure environ aussi longtemps (200 ms) que la zone **V2B** qui forme la voyelle de la syllabe. De plus, le signal recueilli comporte une pseudo-période identique à la pseudo-période du signal émis par l'insert : il s'agit de la réponse soutenue à la fréquence (FFR) dont la périodicité suit fidèlement celle du signal périodique l'ayant évoquée.

La **Fig. 2G** comporte de la même manière deux zones de bruit **B2G1** et **B2G2,** une réponse impulsionnelle **RI2G** et une zone de réponse soutenue à la fréquence **RSF2G**. La réponse impulslonnelle **RI2G** est décelable, d'une part car son amplitude est supérieure à celle du bruit **B2G**, et d'autre part car elle se situe à une latence d'environ 6 ms après la désocclusion à l'origine de cette réponse, ce qui correspond au temps d'arrivée des informations auditives dans le tronc cérébral supérieur. La zone de réponse soutenue à la fréquence **RSF2G** est décelable par le fait qu'elle dure environ aussi longtemps (200 ms) que la zone **V2F** qui forme la voyelle de la syllabe. À nouveau, le signal recueilli comporte une pseudo-période identique à la pseudo-période du signal émis par la prothèse auditive **3** : il s'agit de la réponse soutenue à la fréquence (FFR) dont la périodicité suit fidèlement celle du signal périodique l'ayant évoquée.

Enfin, les **Fig. 2D** et **2H** représentent respectivement les courbes de « PEA » corticaux mesurés à la suite de l'émission de la syllabe /ta/ dans les inserts et dans les prothèses auditives **3**. Ces mesures d'activité électrophysiologique spécifique de type « PEA » corticaux ont été acquises avec un système BrainAmp (BrainProducts) à électrodes actives (actiCAP), à une fréquence d'échantillonnage de 5 kHz, avec une cadence de stimulation de 1 syllabe par seconde. Les mesures ont été enregistrées à l'électrode Cz (placée au vertex), référencée à la moyenne de deux électrodes de recueil placées sur les mastoides. Les réponses corticales sont obtenues à partir de 200 essais élémentaires (stimulation binaurale, polarités alternées), après rejet d'artefact, moyennage, et filtrage passe-bande entre 3 et 80 Hz.

Le début de l'émission du signal dans les inserts est noté **T2D0** et celui de l'émission du signal dans les prothèses **3** est noté **T2H0.** Les courbes présentent des réponses proéminentes notées aux temps **T2D1, T2D2** et **T2D3,,** ainsi que **T2H1, T2H2** et **T2H3**. Les différents pics caractéristiques de la réponse corticale à la syllabe /ta/ présentée **Fig. 2A** et **2E** sont donc présents à des instants sensiblement identiques en comparaison au début de l'émission du signal par les inserts ou par la prothèse auditive : T2H1 = T2D1 + (T2H0 - T2D0) = T2D1 + 67 ms. Plus précisément, chaque évènement transitoire, c'est-à-dire chaque changement rapide du niveau d'énergie acoustique, donne naissance dans le système nerveux auditif à une série d'au moins trois ondes : P50, N100 et P200, la lettre indiquant la polarité (P pour positif ou N pour négatif), et le nombre indiquant la latence en millisecondes. La syllabe /ta/ étant constituée de plusieurs évènements transitoires tels que la désoclusion ou la mise en vibration des cordes vocales, autant de séries de 3 ondes sont évoquées dans le système nerveux auditif central, et se superposent dans les « PEA »corticaux.

Le demandeur a donc vaincu un préjugé de l'homme du métier. Il est possible de faire des mesures de « speech ABR » et de « PEA » corticaux en mettant en œuvre un dispositif comprenant une prothèse auditive à la place d'un écouteur classique de type insert.

La méthode objet de l'invention a pour but d'évaluer l'efficacité de la prothèse **3** en ce sens qu'un réglage adéquat de la prothèse peut aider l'individu **4** à discriminer ou distinguer des unités linguistiques choisies dans la matrice de confusion. La compréhension de la parole par l'individu **4** réside dans sa capacité à discerner les différents phonèmes de la langue qu'il pratique ; c'est là que les différents réglages possibles de la prothèse **3** jouent un rôle primordial.

La méthode objet de l'invention consiste à émettre le stimulus auditif au système nerveux central auditif de l'individu **4** *via* la prothèse **3**. Ce stimulus est composé d'au moins une paire d'unités linguistiques choisies dans une matrice de confusion phonétique. La méthode consiste ensuite à recueillir, dans le module d'enregistrement des données **1.2** de l'unité centrale **1**, les signaux neurophysiologiques générés dans ledit système nerveux central auditif en réponse au stimulus auditif pour chaque unité linguistique au moyen des électrodes **6** et de l'amplificateur EG **5**. Puis les signaux reçus sont traités séparément pour chacune des unités linguistiques puis en comparant au moins un paramètre de la réponse électrophysiologique à ces deux unités linguistiques pour tester l'existence ou non d'une discrimination neurophysiologique entre les différentes unités par le système nerveux central auditif et en évaluant l'efficacité de la prothèse auditive à partir du résultat de cette comparaison.

En pratique, la stimulation est répétée un nombre N de fois compris entre 100 et 6000, préférentiellement entre 1000 et 2000 pour les réponses sous-corticales et au moins 100 pour les réponses corticales. Les deux unités linguistiques sont émises par exemple autant de fois l'une que l'autre, c'est-à-dire N fois chacune. Les unités linguistiques sont préférentiellement émises aléatoirement, et avec des polarités alternées pour minimiser d'éventuels artéfacts de stimulation.

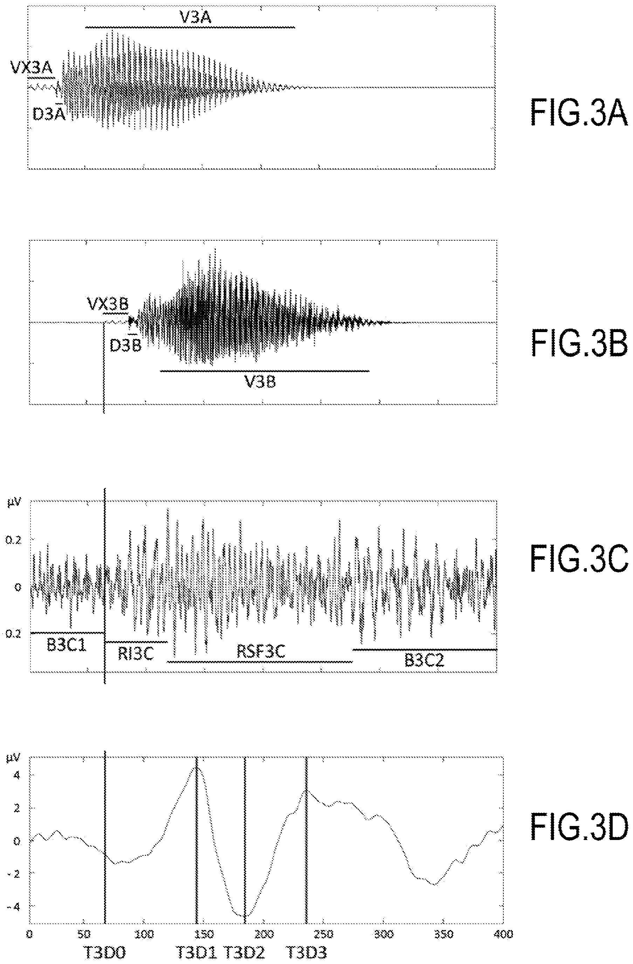

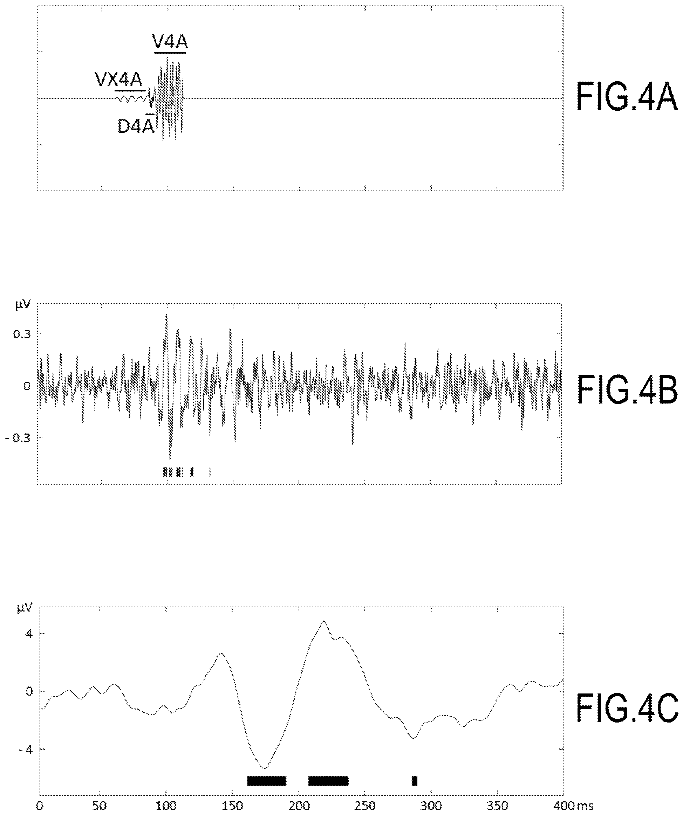

Les **Fig. 3A** à **3H** et **4A** à **4C** représentent un exemple de mise en œuvre de la méthode objet de l'invention pour la comparaison des unités linguistiques /ta/ et /da/.

La **Fig. 3A** représente le signal du son /da/ tel qu'émis par le module de commande **1.1** de l'unité centrale **1**, et la **Fig. 3B** représente le signal du son /da/ tel qu'enregistré en sortie de la prothèse auditive **3**. En particulier, cette syllabe comporte tout d'abord une zone **VX3A**, respectivement **VX3B,** de voisement de durée 25 ms, autrement dit de vibration des cordes vocales durant l'occlusion propre au phonème /d/; puis une désocclusion **D3A**, respectivement **D3B,** de durée 5 ms. La consonne /d/ est donc constituée des éléments suivants : le voisement (mise en vibration des cordes vocales), la désocclusion (retrait du bout de la langue des incisives supérieures), suivie de la reprise très rapide du voisement qui constitue le début de la voyelle /a/. Cette désocclusion **D3A**, respectivement **D3B**, est suivie d'une zone **V3A,** respectivement **V3B,** de voyelle /a/ de durée 200 ms, comportant une pseudo-période de durée 4,6 ms.

La **Fig. 3C** représente les speech ABR mesurées à la suite de l'émission de la syllabe /da/ dans les prothèses auditives **3**. La courbe comporte deux zones de bruit **B3C1** et **B3C2,** une réponse impulslonnelle **RI3C** et une zone de réponse soutenue à la fréquence **RSF3C.** La réponse impulslonnelle **RI3C** est décelable, d'une part car son amplitude est supérieure à celle du bruit **B3C,** et d'autre part car la zone **RI3C** coïncide temporellement avec la zone de désocclusion **D3B,** c'est-à-dire juste après le début de la stimulation sonore (le voisement). La zone de réponse soutenue à la fréquence **RSF3C** est décelable par le fait qu'elle comporte la même durée 200 ms que la voyelle **V3B.** De plus, les ondulations comportent une pseudo-période, suivant fidèlement la pseudo-période du signal émis par la prothèse auditive **3.**

Le début de l'émission du signal du son /da/ est noté **T3D0** pour le temps d'émission de la désocdusion. La courbe des « PEA » corticaux pour la syllabe /da/ présentée via les prothèses auditives **3,** illustrée à la figure **3D****,** présente des réponses proéminentes notées aux temps **T3D1, T3D2** et **T3D3,** correspondant aux ondes P50, N100 et P200.

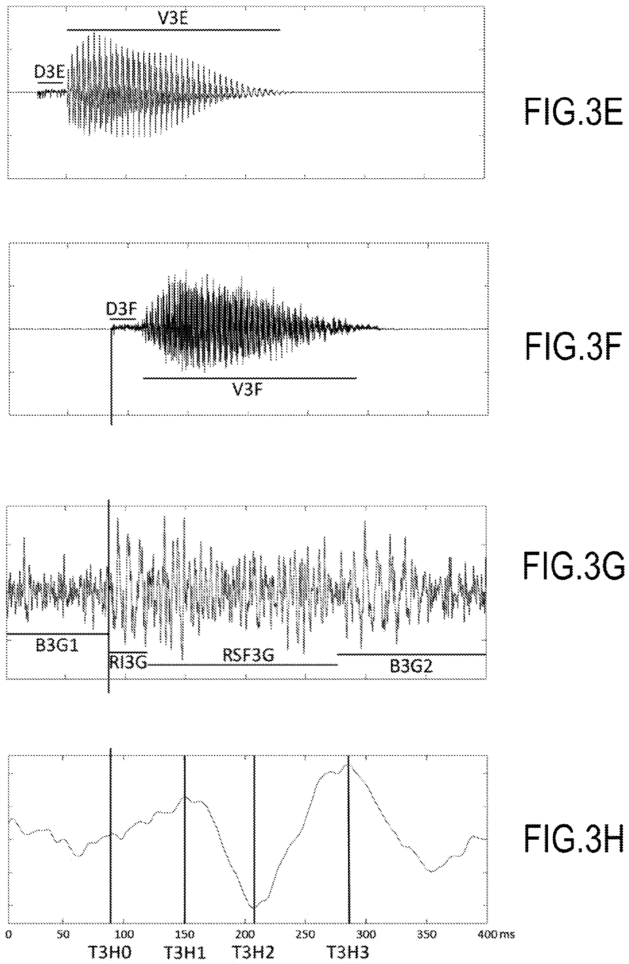

La **Fig. 3E** représente le signal du son /ta/ tel qu'émis par le module de commande **1.1** de l'unité centrale **1,** et la **Fig. 3F** représente le signal du son /ta/ tel qu'enregistré en sortie de la prothèse auditive **3.** En particulier, cette syllabe comporte tout d'abord une zone **D3E** de désocclusion de durée 5 ms, autrement dit de formation de la consonne. Cette zone **D3E** de désocclusion est suivie d'une zone **V3E** de voyelle de durée 200 ms. La zone **V3E** comporte une pseudo-période de même durée que celle de **V3A**, car il s'agit de la même voyelle.

La **Fig. 3G** qui représente le « speech ABR » pour la syllabe /ta/ émise par les prothèses auditives **3,** comporte deux zones de bruit **B3G1** et **B3G2,** une zone **RI3G** de réponse impulsionnelle et une zone de réponse soutenue à la fréquence **RSF3G.** La zone de réponse impulslonnelle **RI3G** est décelable, car son amplitude est supérieure à celle du bruit **B3G1** et **B3G2.** La zone de réponse soutenue à la fréquence **RSF3G** est décelable par le fait qu'elle comporte la même durée 200 ms que la zone **V3F** qui forme la voyelle de la syllabe. De plus, les ondulations comportent une pseudo-période, la même que la pseudo-période du signal émis par la prothèse auditive **3.**

Le début de l'émission du signal du son /ta/ est noté **T3H0** pour le temps de la désocdusion. La courbe des « PEA corticaux » pour la syllabe /ta/ émise par les prothèses auditives **3** est illustrée à la figure **3H** et présente des réponses proéminentes notées aux temps **T3H1, T3H2** et **T3H3**, correspondant aux ondes P50, N100 et P200.

Pour comparer les signaux électrophysiologiques correspondant aux unités linguistiques /ta/ et /da/, une ou plusieurs des étapes suivantes peuvent être mises en œuvre. En effet il est possible, après les étapes de prétraitement (rejet d'artefact, filtrage, moyennage, autres étapes de débruitage) permettant d'obtenir les courbes de speech ABR et/ou de **«PEA»** corticaux :
- d'extraire des latences de pics, des amplitudes de pics, des différences de latence entre pics, des pentes de la courbe entre deux pics, ou des aires sous une partie de la courbe ;
- de rechercher dans des formes d'onde et/ou des décompositions temps-fréquence, une réponse attendue spécifiquement pour l'une desdites unités linguistiques ;
- de comparer directement des formes d'ondes de chaque signal neurophysiologique recueilli en réponse par soustraction ;
- de comparer des décompositions temps-fréquence.

Par exemple, pour comparer directement les formes d'ondes de chaque signal, une soustraction peut être mise en œuvre. La **Fig. 4A** représente la soustraction des signaux /da/ et /ta/ délivrés par l'ordinateur **1** *via* la prothèse **3** à l'individu **4**, ces signaux /da/ et /ta/ sont illustrés respectivement **Fig. 3A** et **3E****.** En particulier, la **Fig. 4A** met en évidence la différence acoustique entre les unités linguistiques /da/ et /ta/. Ces différences se situent dans la partie initiale de l'unité linguistique : voisement **VX4A,** désocclusion **D4A,** et début de la voyelle **V4A**. La composition fréquentlelle du signal change au cours de la syllabe, et la composition fréquentielle du début de la voyelle dépend de la consonne qui la précède : c'est ce que montre la zone **V4A.** En revanche la voyelle est identique dans les deux syllabes **(V3A** et **V3F).**

La **Fig. 4B** représente la soustraction des mesures de speech ABR telles que représentées en **Fig. 3C** et **3G** tandis que la **Fig. 4C** représente la soustraction des mesures de **«PEA»** corticaux telles que représentées en **Fig. 3D** et **3H**. Il a été effectué un test statistique de Wilcoxon sur les N répétitions, échantillon temporel par échantillon temporel, avec p<0.05, corrigé pour les tests multiples par FDR (« False Discovery Rate »), cette procédure statistique permettant de contrôler le taux de « faux positifs » à travers l'ensemble des tests statistiques réalisés (ici, un par échantillon temporel, soit 10000 tests pour les speech ABR, échantillonnés à 25000 Hz, **Fig. 4B****,** et 2000 tests pour les **«PEA»** corticaux échantillonnés à 5000 Hz, **Fig. 4C****).**

Ainsi, les échantillons temporels pour lesquels les signaux électrophysiologiques sont significativement différents entre les deux unités linguistiques sont mis en évidence par les barres noires sous les courbes. Cela signifie qu'en affirmant que ces zones de signal sont différentes pour les deux stimuli, on a moins de 5 % de chance de se tromper (pour p<0.05).

En particulier, la **Fig. 4B** met en évidence la discrimination neurophysiologique des syllabes /da/ et /ta/ au niveau des « speech ABR » car il existe des différences statistiquement significatives entre les réponses électrophysiologiques engendrées par les deux unités linguistiques, comme le montrent les barres noires dans les 30 ms qui suivent le début de la voyelle. De même, la **Fig. 4C** montre la discrimination neurophysiologique des syllabes /da/ et /ta/ au niveau des **«PEA»** corticaux car il y a des différences à des latences correspondant aux ondes P50 et N100 évoquées par le début des sons. Ces résultats indiquent qu'il y a bien une discrimination neurophysiologique entre /da/ et /ta/, c'est-à-dire un traitement approprié par le système nerveux auditif des différences entre les deux consonnes et en particulier du voisement présent seulement pour le /da/. Selon un mode de mise en œuvre de l'invention, il peut être envisagé de recueillir en tant que signaux électrophysiologiques, uniquement les signaux de « Speech ABR » et de traiter de tels signaux permettant d'évaluer de façon pertinente l'efficacité d'une prothèse auditive. Selon un autre mode de mise en œuvre de l'invention, il peut être prévu de recueillir les signaux « Speech ABR » et de « PEA » corticaux pour ensuite être traitée comme décrit ci-dessus,

La **Fig. 5** représente une matrice de confusion phonétique en anglais et met en évidence trois types de confusions particulières :
- sur la présence ou non d'une consonne finale, par exemple « rope » et« row » ;
- sur la consonne initiale, par exemple « cook » et « took », et
- sur la présence ou non d'un « s » Initiai, par exemple « ski » et « key ».

La **Fig. 6** est un acougramme phonétique représentant les diverses zones fréquentielles de chaque consonne en français. Le chevauchement de ces zones fréquentielles explique chez le sujet malentendant et/ou porteur d'une prothèse auditive mal réglée la plupart des confusions phonétiques. Les confusions les plus fréquentes portent sur les paires de consonnes suivantes : /p/-/t/, /k/-/t/, /f/-/s/, /∫/-/s/, /b/-/d/, et /m/-/n/.

Enfin, la méthode d'évaluation de l'efficacité de prothèse auditive objet de l'invention permet de changer au moins un paramètre de réglage de ladite prothèse auditive. En effet, en utilisant l'information d'un acougramme phonétique par exemple, on peut déterminer le ou les traits acoustiques dont le traitement par ledit système nerveux auditif est insuffisant, et on ainsi changer les paramètres de réglage de la prothèse auditive pour permettre ladite discrimination neurophysiologique des unités linguistiques émises.

Par exemple, si le /da/ et le /ta/ ne sont pas discriminés par l'individu **4,** cela signifie qu'il ne perçoit pas la différence de voisement ce qui est dû à une perte auditive dans les basses fréquences, c'est-à-dire pour des fréquences comprises entre 100 Hz à 300 Hz. En conséquence, le paramètre de la prothèse à changer est le gain de la plage de fréquences allant de 100 Hz à 300 Hz.

Dans certaines pertes auditives notamment la presbyacousie, l'audition sur les fréquences graves est relativement préservée. Pour cette raison, les fréquences graves ne sont habituellement pas (ou peu) amplifiées par la prothèse auditive, et elles parviennent au système nerveux auditif uniquement via cette audition préservée dans les graves. Il est cependant indispensable que le système nerveux auditif puisse coder au mieux l'ensemble des composantes spectrales de la parole, fréquences graves incluses, afin de permettre au porteur de prothèse auditive de comprendre un locuteur. Le procédé de recueil de speech ABR ci-dessus, en délivrant directement des sons de parole *via* la prothèse auditive, repose sur l'administration de niveaux sonores définis par les paramètres de réglage de ladite prothèse auditive. Le sujet testé est ainsi privé de l'audition naturelle des fréquences graves. En stimulant directement via la prothèse, ledit procédé prévoit en conséquence de délivrer un signal enrichi en basses fréquences comparativement à l'amplification qui serait appliquée en situation naturelle, pour ajouter au signal d'entrée l'information acoustique qui parviendrait en situation d'écoute quotidienne via l'audition préservée dans les graves. Cette précaution méthodologique conditionne formellement la transcription des caractéristiques spectrales des sons de parole dans le signal speech ABR recueilli grâce audit procédé conforme à l'invention.

L'invention n'est pas limitée aux exemples décrits et représentés car diverses modifications peuvent y être apportées sans sortir de son cadre.

## Revendications

1. Méthode d'évaluation de l'efficacité d'une prothèse auditive (**3**) consistant :
- à émettre un stimulus *via* une prothèse auditive (**3**), délivrant ainsi un stimulus auditif au système nerveux auditif d'une personne équipée de ladite prothèse auditive ;
- à recueillir, en tant que signaux électrophysiologiques, les signaux neurophysiologiques émis par ledit système nerveux auditif en réponse au stimulus auditif, au moyen d'un système d'électro- ou magnétoencéphalographie, et
- à traiter les signaux électrophysiologiques reçus ;
la méthode consistant :
- à émettre un stimulus composé d'au moins une paire d'unités linguistiques choisies dans une matrice de confusion phonétique ;
- à recueillir séparément les signaux électrophysiologiques en réponse à chaque unité linguistique administrée via la prothèse auditive (3), et
- à traiter les signaux :
• en comparant les signaux électrophysiologiques correspondant aux différentes unités linguistiques testées en effectuant une ou plusieurs des étapes suivantes, consistant :
➢ à extraire des latences de pics, des amplitudes de pics, des différences de latence entre pics, des pentes de la courbe entre deux pics, ou des aires sous une partie de la courbe ;
➢ à rechercher dans des formes d'onde et/ou des décompositions temps-fréquence, une réponse attendue spécifiquement pour l'une desdites unités linguistiques ;
➢ à comparer directement des formes d'ondes de chaque signal neurophysiologique recueilli en réponse par soustraction ;
➢ à comparer des décompositions temps-fréquence et
• en évaluant l'efficacité de la prothèse auditive à partir du résultat de cette comparaison entre les signaux électrophysiologiques correspondant aux différentes unités linguistiques testées ;
**caractérisée en ce qu'**elle consiste à recueillir simultanément en tant que signaux électrophysiologiques, des signaux de « Speech Auditory Brainstem Response » (« speech ABR ») et de Potentiels Evoqués Auditifs (« PEA ») corticaux.

2. Méthode d'évaluation de l'efficacité d'une prothèse auditive selon la revendication 1, **caractérisée en ce qu'**elle consiste à choisir les unités linguistiques parmi les phonèmes, tonèmes ou chronèmes, ou leurs assemblages en syllabes.

3. Méthode d'évaluation de l'efficacité d'une prothèse auditive selon l'une des revendications précédentes, **caractérisée en ce qu'**elle consiste à identifier, en fonction du résultat dudit traitement des signaux, au moins un trait acoustique dudit stimulus dont le traitement par ledit système nerveux auditif est insuffisant pour permettre ladite discrimination neurophysiologique desdites unités linguistiques émises.

4. Méthode d'évaluation de l'efficacité d'une prothèse auditive selon la revendication 3, **caractérisée en ce qu'**elle consiste à changer au moins un paramètre de réglage de ladite prothèse auditive en fonction dudit trait acoustique dont le traitement par ledit système nerveux auditif est insuffisant pour permettre ladite discrimination neurophysiologique desdites unités linguistiques émises.

5. Méthode d'évaluation de l'efficacité d'une prothèse auditive selon l'une des revendications précédentes, **caractérisée en ce qu'**elle consiste à transmettre le stimulus à la prothèse par une liaison sans fil.

## Patentansprüche

1. Verfahren zur Bewertung der Wirksamkeit eines Hörgeräts (3), das besteht aus:
- Senden eines Stimulus über ein Hörgerät (3), wodurch ein Hörstimulus an das Hörnervensystem einer mit dem Hörgerät ausgestatteten Person geliefert wird,
- Sammeln der von dem Hörnervensystem als Reaktion auf den Hörstimulus gesendeten neurophysiologischen Signale als elektrophysiologische Signale mittels eines Elektro- oder Magnet-Enzephalographiesystems, und
- Verarbeiten der empfangenen elektrophysiologischen Signale,
wobei das Verfahren besteht aus:
- Senden eines Stimulus, der aus mindestens einem Paar Spracheinheiten zusammengesetzt ist, die in einer phonetischen Verwechslungsmatrix ausgewählt werden,
- getrenntes Sammeln der elektrophysiologischen Signale als Reaktion auf jede über das Hörgerät (3) verabreichte Spracheinheit, und
- Verarbeiten der Signale:
• durch Vergleichen der elektrophysiologischen Signale, die den verschiedenen getesteten Spracheinheiten entsprechen, durch Durchführen von einem oder mehreren der folgenden Schritte, die bestehen aus:
➢ Extrahieren von Spitzenlatenzen, von Spitzenamplituden, von Latenzdifferenzen zwischen Spitzen, von Steigungen der Kurve zwischen zwei Spitzen oder von Flächen unter einem Teil der Kurve,
➢ Suchen einer spezifisch für eine der Spracheinheiten erwarteten Reaktion in Wellenformen und/oder Zeit-Frequenz-Analysen,
➢ direktes Vergleichen der Wellenformen jedes als Reaktion gesammelten neurophysiologischen Signals durch Subtraktion,
➢ Vergleichen von Zeit-Frequenz-Analysen und
• durch Bewerten der Wirksamkeit des Hörgeräts ausgehend von dem Ergebnis dieses Vergleichs zwischen den elektrophysiologischen Signalen, die den verschiedenen getesteten Spracheinheiten entsprechen,
**dadurch gekennzeichnet, dass** es im gleichzeitigen Sammeln der Signale von "Speech Auditory Brainstem Response" ("Speech ABR") und von kortikalen akustisch evozierten Potentialen ("AEP") als elektrophysiologische Signale besteht.

2. Verfahren zur Bewertung der Wirksamkeit eines Hörgeräts nach Anspruch 1, **dadurch gekennzeichnet, dass** es im Wählen der Spracheinheiten unter Phonemen, Tonemen oder Chronemen oder ihrer Verbindungen in Silben besteht.

3. Verfahren zur Bewertung der Wirksamkeit eines Hörgeräts nach einem der vorhergehenden Ansprüche, **dadurch gekennzeichnet, dass** es im Identifizieren mindestens eines akustischen Merkmals des Stimulus, dessen Verarbeitung durch das Hörnervensystem unzureichend ist, in Abhängigkeit von dem Ergebnis der Verarbeitung der Signale besteht, um die neurophysiologische Unterscheidung der gesendeten Spracheinheiten zu ermöglichen.

4. Verfahren zur Bewertung der Wirksamkeit eines Hörgeräts nach Anspruch 3, **dadurch gekennzeichnet, dass** es im Ändern mindestens eines Regelungsparameters des Hörgeräts in Abhängigkeit von dem akustischen Merkmal, dessen Verarbeitung durch das Hörnervensystem unzureichend ist, besteht, um die neurophysiologische Unterscheidung der gesendeten Spracheinheiten zu ermöglichen.

5. Verfahren zur Bewertung der Wirksamkeit eines Hörgeräts nach einem der vorhergehenden Ansprüche, **dadurch gekennzeichnet, dass** es im Senden des Stimulus an die Prothese über eine drahtlose Verbindung besteht.

## Claims

1. A method for evaluating the effectiveness of an auditory prosthesis (3) consisting in:
- transmitting a stimulus via an auditory prosthesis (3), thereby delivering an auditory stimulus to the auditory nervous system of a person fitted with said auditory prosthesis;
- picking up, as electrophysiological signals, the neurophysiological signals transmitted by said auditory nervous system in response to the auditory stimulus, by means of an electro- or magnetoencephalography system, and
- processing the received electrophysiological signals;
the method consisting in:
- transmitting a stimulus made up of at least one pair of linguistic units selected from a phonetic confusion matrix;
- picking up separately the electrophysiological signals in response to each linguistic unit administered via the auditory prosthesis (3), and
- processing the signals by:
• comparing the electrophysiological signals corresponding to the different tested linguistic units by performing one or more of the following steps, consisting in:
➢ extracting peak latencies, peak amplitudes, peak-to-peak latency differences, slopes of the curve between two peaks, or areas under a portion of the curve;
➢ searching waveforms and/or time-frequency decompositions for a response specifically expected for one of said linguistic units;
➢ directly comparing the waveforms of each neurophysiological signal picked up in response by subtraction;
➢ comparing time-frequency decompositions and
• evaluating the effectiveness of the auditory prosthesis based on the result of this comparison between the electrophysiological signals corresponding to the different linguistic units tested;
**characterized in that** it consists in simultaneously picking as electrophysiological signals, signals of speech auditory brainstem response (speech ABR) and of cortical auditory evoked potentials (AEP).

2. The method for evaluating the effectiveness of an auditory prosthesis as claimed in claim 1, **characterized in that** it consists in selecting linguistic units from phonemes, tonemes or chronemes, or assemblies thereof as syllables.

3. The method for evaluating the effectiveness of an auditory prosthesis as claimed in one of the preceding claims, **characterized in that** it consists in identifying, as a function of the result of said signal processing, at least one acoustic feature of said stimulus whose processing by said auditory nervous system is insufficient to allow said neurophysiological discrimination of said transmitted linguistic units.

4. The method for evaluating the effectiveness of an auditory prosthesis as claimed in claim 3, **characterized in that** it consists in changing at least one setting parameter of said auditory prosthesis as a function of said acoustic feature whose processing by said auditory nervous system is insufficient to allow said neurophysiological discrimination of said transmitted linguistic units.

5. The method for evaluating the effectiveness of an auditory prosthesis as claimed in one of the preceding claims, **characterized in that** it consists in transmitting the stimulus to the prosthesis via a wireless link.
